(19) Europäisches Patentamt
European Patent Office
Office européen des brevets

(11) **EP 4 390 965 A1**

(12) **EUROPEAN PATENT APPLICATION**

(43) Date of publication:
**26.06.2024 Bulletin 2024/26**

(51) International Patent Classification (IPC):
***G16H 50/20*** (2018.01)

(21) Application number: **23219076.9**

(22) Date of filing: **21.12.2023**

(52) Cooperative Patent Classification (CPC):
**G16H 50/20;** G16H 10/40

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 US 202263477032 P**

(71) Applicants:
• **Kaohsiung Medical University**
  **Kaohsiung 80708 (TW)**
• **National Sun Yat-Sen University**
  **Gushan District, Kaohsiung City 804 (TW)**

(72) Inventors:
• **CHEN, Hao-Wei**
  **807 Kaohsiung City (TW)**
• **KAO, Chung-Yao**
  **800 Kaohsiung City (TW)**
• **LEE, Jung-Ting**
  **800 Kaohsiung City (TW)**
• **CHEN, Yu-Chen**
  **806 Kaohsiung City (TW)**
• **WEI, Pei-Siou**
  **434 Taichung City (TW)**

(74) Representative: **f & e patent**
  **Braunsberger Feld 29**
  **51429 Bergisch Gladbach (DE)**

(54) **METHOD FOR ESTABLISHING MODEL TO DETERMINE WHETHER A SUBJECT HAS NEPHROLITHIASIS**

(57) A method for establishing a model to determine whether a subject has nephrolithiasis includes: grouping training data sets into a number N of preliminary groups, where N is a positive integer; obtaining a number N of preliminary models based on the preliminary groups; averaging the preliminary models to obtain an average model; and obtaining a prediction model for determining whether the subject has nephrolithiasis based on the average model.

FIG. 1

**Description**

The disclosure relates to a method for establishing a model to determine whether a subject has nephrolithiasis, and more particularly to a method for establishing a model to determine whether a subject has nephrolithiasis by using machine learning (ML) methodologies.

Conventionally, the diagnosis of nephrolithiasis (commonly known as kidney stones) is performed based on technologies of medical imaging, such as renal ultrasonography, abdominal computed tomography (CT) or the like.

According to an aspect of the disclosure, there is provided a method for establishing a model to determine whether a subject has nephrolithiasis according to claim 1.

Other features and advantages of the disclosure will become apparent in the following detailed description of the embodiment(s) with reference to the accompanying drawings. It is noted that various features may not be drawn to scale.

Figure 1 is a flow chart illustrating a method for establishing a model to determine whether a subject has nephrolithiasis according to an embodiment of the disclosure.

Figure 2 is a schematic diagram illustrating one of non-output layers of a multi-layer fully connected neural network.

Figure 3 is a schematic diagram illustrating an output layer of the multi-layer fully connected neural network.

Figure 4 is a schematic diagram illustrating construction of a prediction model.

Figures 5-9 are graphs respectively illustrating receiver operating characteristic (ROC) curves which are respectively obtained by using ANN, LR, KNN, SVM and RF models.

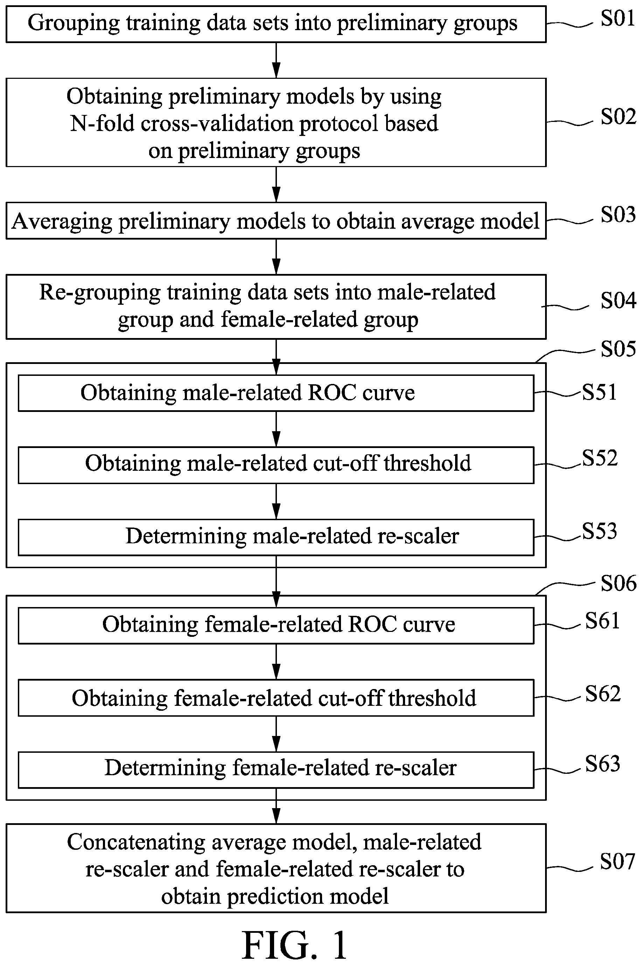

Referring to Figure 1, an embodiment of a method for establishing a model to determine whether a subject (e.g., a patient) has nephrolithiasis (i.e., kidney stones) according to the disclosure is illustrated. Within a machine learning (ML) framework, the method is implemented by using the Python programming language and the Scikit-learn software library (version 1.1.3), and techniques of artificial neural network (ANN) and deep learning (DL) are adopted to develop the model. Since developing a model by techniques of ANN and DL has been well known to one skilled in the relevant art, detailed explanation of the same is omitted herein for the sake of brevity. The method includes steps S01 to S07 delineated below.

In step S01, a plurality of training data sets that are respectively related to a plurality of patients are grouped into a number N of preliminary groups, where N is a positive integer. In this embodiment, N is ten, but N is not limited to the disclosure herein and may vary in other embodiments.

Each of the training data sets includes eight parameters, i.e., a gender indicator that indicates gender of the corresponding patient (e.g., one for male and zero for female), age of the corresponding patient, a body mass index (BMI) that is related to the corresponding patient, an estimated glomerular filtration rate (eGFR) that is related to the corresponding patient, a value of urine pH that is related to the corresponding patient, a gout indicator that indicates whether the corresponding patient was ever diagnosed with gout (e.g., one for those who have once been diagnosed with gout and zero for those who were never diagnosed with gout), a diabetes mellitus (DM) indicator that indicates whether the corresponding patient was ever diagnosed with DM (e.g., one for those who have once been diagnosed with DM and zero for those who were never diagnosed with DM), and a bacteriuria indicator that indicates whether the corresponding patient was ever diagnosed with bacteriuria (e.g., one for those who have once been diagnosed with bacteriuria and zero for those who were never diagnosed with bacteriuria). The eGFR is calculated by using the isotope dilution mass spectrometry traceable Modification of Diet in Renal Disease formula (hereinafter also referred to as the IDMS-traceable MDRD formula) that is expressed as:

$$\text{eGFR } [mL/min/1.73m^2] = 175 \times (Scr)^{-1.154} \times (Age)^{-0.203} \times (0.742)^G,$$

where $[mL/min/1.73m^2]$ represents a unit of eGFR, $Scr$ represents a blood creatinine concentration that is related to the corresponding patient, $Age$ represents the age of the corresponding patient, and G represents the gender of the corresponding patient. Specifically, the variable G has a value of one when the gender indicator indicates that the corresponding patient is female, and has a value of zero when the gender indicator indicates that the corresponding patient is male. It is worth to note that in some embodiments, the IDMS-traceable MDRD formula further adopts a race multiplier for considering race of the corresponding patient (e.g., adopts a multiplier of 1.212 when the corresponding patient is an African American or a Black). That is to say, the IDMS-traceable MDRD formula for an African American or a Black would be expressed as:

$$\text{eGFR } [mL/min/1.73m^2] = 175 \times (Scr)^{-1.154} \times (Age)^{-0.203} \times (0.742)^G \times 1.212.$$

In one embodiment, each of the training data sets includes ten parameters, i.e., a number of red blood cells in a urine sample of the corresponding patient, a blood creatinine concentration that is related to the corresponding patient, the gender indicator that indicates gender of the corresponding patient, the age of the corresponding patient, the BMI that is related to the corresponding patient, the eGFR that is related to the corresponding patient, the value of urine pH that is related to the corresponding patient, the gout indicator that indicates whether the corresponding patient was ever diagnosed with gout, the DM indicator that indicates whether the corresponding patient was ever diagnosed with DM, and the bacteriuria indicator that indicates whether the corresponding patient was ever diagnosed with bacteriuria.

In one embodiment, each of the training data sets includes only three parameters, i.e., the gender indicator that indicates gender of the corresponding patient, the age of the corresponding patient and the eGFR that is related to the corresponding patient.

In one embodiment, each of the training data sets includes only three parameters, i.e., the gender indicator that indicates gender of the corresponding patient, the age of the corresponding patient and the blood creatinine concentration that is related to the corresponding patient.

In one embodiment, each of the training data sets includes only three parameters, i.e., the gender indicator that indicates gender of the corresponding patient, the age of the corresponding patient and the number of red blood cells in a urine sample of the corresponding patient.

It is worth noting that the training data sets are grouped into the number N of preliminary groups at random. In some embodiments, the training data sets are grouped into the number N of preliminary groups in a manner that the training data sets are evenly grouped to decrease statistical differences among the number N of preliminary groups; that is to say, for each of the above-mentioned parameters, statistical values related to the same parameter respectively for the number N of preliminary groups are not much different from each other, and the statistical value for each of the number N of preliminary groups is calculated based on all values of the parameter respectively in those of the training data sets that are included in the preliminary group (e.g., an average). In particular, firstly, a mean and a standard deviation related to each of the above-mentioned parameters (hereinafter also referred to as eight parameters) are calculated based on all of the training data sets. Secondly, for each of the training data sets, a sum of squares (hereinafter also referred to as an SS) respectively of z-scores respectively of the eight parameters is calculated, i.e.,

$$\sum_{j=1}^{8} z_j^2 = \sum_{j=1}^{8} \left(\frac{p_j - \mu_j}{\sigma_j}\right)^2$$

, where $z_j$ represents the z-score of a $j^{th}$ one of the eight parameters, $p_j$ represents the value of the $j^{th}$ one of the eight parameters of the training data set, $\mu_j$ represents the mean of all values of the $j^{th}$ one of the eight parameters that is calculated based on all of the training data sets, and $\sigma_j$ represents the standard deviation of all values of the $j^{th}$ one of the eight parameters that is calculated based on all of the training data sets. Thirdly, the training data sets are sorted, and thus are arranged in an ascending order (or a descending order in other embodiments) of the SSs of the training data sets. Fourthly, a grouping procedure is implemented by respectively assigning first to $N^{th}$ ones of the training data sets that have been sorted and that have not been assigned yet to the number N of preliminary groups, and the grouping procedure is repeated until all of the training data sets have been assigned. That is to say, at a first implementation of the grouping procedure, a first number N of the training data sets that respectively have a first greatest number N of the SSs are respectively assigned to the number N of preliminary groups; at a second implementation of the grouping procedure, a second number N of the training data sets that respectively have a second greatest number N of the SSs are respectively assigned to the number N of preliminary groups; and a similar procedure is followed until all of the training data sets have been assigned. It should be noted that at the last implementation of the grouping procedure, a number of the training data sets that are to be assigned may be less than the number N.

In step S02, a number N of preliminary models are obtained by using N-fold cross-validation protocol based on the preliminary groups. Since the N-fold cross-validation protocol has been well known to one skilled in the relevant art, detailed explanation of the same is omitted herein for the sake of brevity and only a brief explanation is provided herein. At an $i^{th}$ iteration, where i is an integer ranging from one to N, an $i^{th}$ one of the number N of preliminary groups is withheld for internal validation of an $i^{th}$ one of the number N of preliminary models, while remaining ones of the number N of preliminary groups are used for training the $i^{th}$ one of the number N of preliminary models. That is to say, at a first iteration, a first one of the number N of preliminary groups is not used for training a first one of the number N of preliminary models and is withheld for internal validation of the first one of the number N of preliminary models, while remaining ones of the number N of preliminary groups are used for training the first one of the number N of preliminary models; at a second iteration, a second one of the number N of preliminary groups is not used for training a second one of the number N of preliminary models and is withheld for internal validation of the second one of the number N of preliminary models, while remaining ones of the number N of preliminary groups are used for training the second one of the number N of preliminary models; and a similar procedure is followed for third to $(N-1)^{th}$ iterations, and at an $N^{th}$ iteration, an $N^{th}$ one of the number N of preliminary groups is not used for training an $N^{th}$ one of the number N of preliminary models and is withheld for internal validation of the $N^{th}$ one of the number N of preliminary models, while remaining ones of the number N of preliminary groups are used for training the N$^{th}$ one of the number N of preliminary models.

Each of the preliminary models is expressed as $M_i(x) = \frac{1}{1+e^{f_i(x)}}$ , i = 1,2, ..., N, where $M_i$ represents the preliminary model, x represents an input of the preliminary model, and $f_i(x)$ is a nonlinear function that is obtained by using a multi-layer fully connected neural network. Each of the preliminary models has a plurality of trainable variables that are optimized by using an Adam optimizer with preset parameters and a cross-entropy loss function. Specifically, while training each of the preliminary models, the cross-entropy loss function measures a difference between an ideal output (i.e., the ground truth) and a predicted output of the preliminary model, and the Adam optimizer is utilized to adjust the trainable variables of the preliminary model. Training the preliminary model and adjusting the trainable variables of the preliminary model are repeated until the cross-entropy loss function converges to a local minimum, at which time the trainable variables of the preliminary model are optimized.

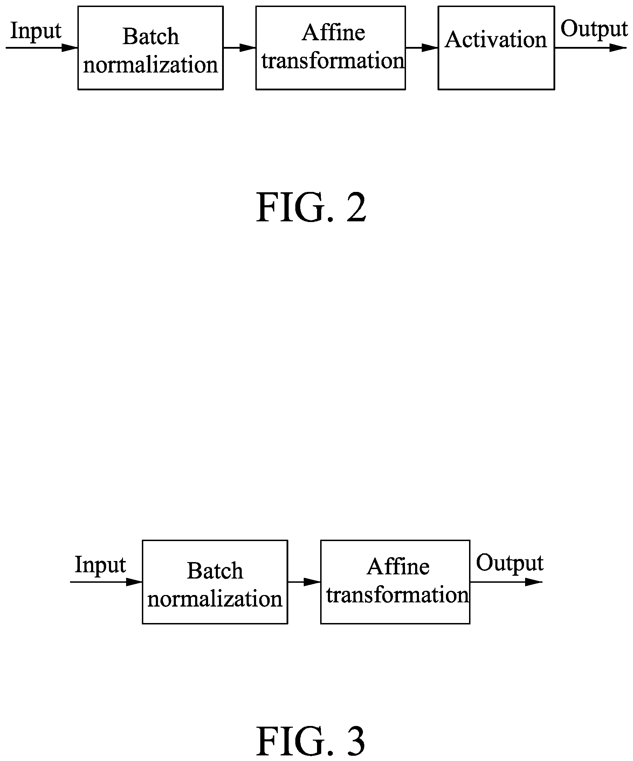

The multi-layer fully connected neural network has a plurality of non-output layers and an output layer, and is expressed as $\frac{1}{N} \times \sum_{i=1}^{N} M_i(x)$ . Referring to Figure 2, each of the non-output layers consists of three concatenated components respectively for batch normalization, affine transformation and activation (i.e., an output of the component for batch normalization serves as an input of the component for affine transformation, and an output of the component for affine transformation serves as an input of the component for activation). Referring to Figure 3, the output layer consists of two concatenated components respectively for batch normalization and affine transformation. Since batch normalization, affine transformation and activation have been well known to one skilled in the relevant art, detailed explanation of the same is omitted herein for the sake of brevity. Particularly, in this embodiment, the multi-layer fully connected neural network has eight of the non-output layers connected in sequence. A first one of the non-output layers in the sequence has an input dimension (i.e., a number of inputs) of 8, an output dimension (i.e., a number of outputs) of 16, and 160 trainable variables, and performs activation based on a rectified linear unit (ReLU) activation function; a second one of the non-output layers in the sequence has an input dimension of 16, an output dimension of 32, and 576 trainable variables, and performs activation based on a ReLU activation function; a third one of the non-output layers in the sequence has an input dimension of 32, an output dimension of 64, and 2176 trainable variables, and performs activation based on a ReLU activation function; a fourth one of the non-output layers in the sequence has an input dimension of 64, an output dimension of 64, and 4288 trainable variables, and performs activation based on a ReLU activation function; a fifth one of the non-output layers in the sequence has an input dimension of 64, an output dimension of 128, and 8448 trainable variables, and performs activation based on a sigmoid function; a sixth one of the non-output layers in the sequence has an input dimension of 128, an output dimension of 64, and 8512 trainable variables, and performs activation based on a ReLU activation function; a seventh one of the non-output layers in the sequence has an input dimension of 64, an output dimension of 32, and 2208 trainable variables, and performs activation based on a ReLU activation function; and an eighth one of the non-output layers in the sequence has an input dimension of 32, an output dimension of 16, and 592 trainable variables, and performs activation based on a ReLU activation function. The output layer has an input dimension of 16, an output dimension of 1, and 49 trainable variables. Therefore, each of the preliminary models has 27009 trainable variables in total. Constitution of the multi-layer fully connected neural network is summarized in Table 1 below.

Table 1

| Layer | | Input Dimension | Output Dimension | Activation Function | Number of Trainable Variables |
|---|---|---|---|---|---|
| Non-Output | 1 | 8 | 16 | ReLU | 160 |
| | 2 | 16 | 32 | ReLU | 576 |
| | 3 | 32 | 64 | ReLU | 2176 |
| | 4 | 64 | 64 | ReLU | 4288 |
| | 5 | 64 | 128 | Sigmoid | 8448 |
| | 6 | 128 | 64 | ReLU | 8512 |
| | 7 | 64 | 32 | ReLU | 2208 |
| | 8 | 32 | 16 | ReLU | 592 |
| Output | | 16 | 1 | N/A | 49 |

In step S03, the preliminary models are averaged to obtain an average model. It is worth to note that such approach is commonly known as ensemble averaging. Since model averaging has been well known to one skilled in the relevant art, detailed explanation of the same is omitted herein for the sake of brevity.

In step S04, the training data sets are re-grouped into a male-related group and a female-related group. The male-related group includes those of the training data sets that are related to male patients, and the female-related group includes those of the training data sets that are related to female patients.

In step S05, a male-related re-scaler is determined. Specifically, step S05 includes sub-steps S51 to S53. In sub-step S51, the average model is applied to those of the training data sets that are included in the male-related group to obtain a plurality of male-related outputs, and then a male-related receiver operating characteristic (ROC) curve is obtained based on the male-related outputs. In sub-step S52, a male-related cut-off threshold is determined based on a Youden's index of the male-related ROC curve. Since plotting an ROC curve and determining a cut-off threshold based on a Youden's index of the ROC curve have been well known to one skilled in the relevant art, detailed explanation of the same is omitted herein for the sake of brevity. In sub-step S53, the male-related re-scaler is determined based on the male-related cut-off threshold. The male-related re-scaler is expressed as

$$S_m(y_m) = \frac{0.5}{T_m} \times y_m + max\{0, (y_m - T_m)\} \times \left(\frac{0.5}{1-T_m} - \frac{0.5}{T_m}\right)$$

, where $S_m$ represents the male-related re-scaler, $y_m$ represents an output of the average model, which is inputted to the male-related re-scaler, $T_m$ represents the male-related cut-off threshold, and $max\{a, b\}$ is an operation for obtaining the greater of a value $a$ and a value $b$, where the value $a$ and the value $b$ are arbitrary values.

In step S06, a female-related re-scaler is determined. Specifically, step S06 includes sub-steps S61 to S63. In sub-step S61, the average model is applied to those of the training data sets that are included in the female-related group to obtain a plurality of female-related outputs, and then a female-related ROC curve is obtained based on the female-related outputs. In sub-step S62, a female-related cut-off threshold is determined based on a Youden's index of the female-related ROC curve. In sub-step S63, the female-related re-scaler is determined based on the female-related cut-off threshold. The female-related re-scaler is mathematically expressed as

$$S_f(y_f) = \frac{0.5}{T_f} \times y_f + max\{0, (y_f - T_f)\} \times \left(\frac{0.5}{1-T_f} - \frac{0.5}{T_f}\right)$$

, where $S_f$ represents the female-related re-scaler, $y_f$ represents an output of the average model, which is inputted to the female-related re-scaler, and $T_f$ represents the female-related cut-off threshold.

Due to the difference in physiological parameters between males and females, the male-related re-scaler and the female-related re-scaler are used to unify and normalize the outputs of the average model, such that a common threshold (e.g., 0.5) can be devised for determining whether a subject of either gender has nephrolithiasis, where an output of either the male-related re-scaler or the female-related re-scaler, depending upon the gender of the subject, is to be compared with the common threshold for making the diagnosis.

It is worth to note that step S05 for determining the male-related re-scaler and step S06 for determining the female-related re-scaler are interchangeable in order. That is to say, in some embodiments, step S06 is executed prior to executing step S05. Alternatively, in some embodiments, steps S05 and S06 may be executed simultaneously.

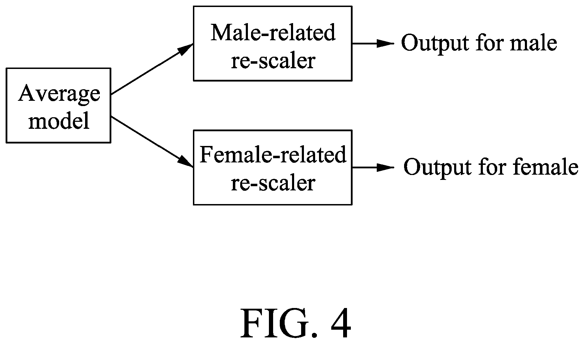

In step S07, the average model, the male-related re-scaler and the female-related re-scaler are concatenated to obtain a prediction model for determining whether the subject has nephrolithiasis. Particularly, the male-related re-scaler and the female-related re-scaler are connected in parallel, and the average model and the parallel connection of the male-related re-scaler and the female-related re-scaler are connected as shown in Figure 4. Further, a comparison between the common threshold (e.g., 0.5) and the output of the male-related re-scaler is made for determining whether a male subject has nephrolithiasis, and a comparison between the common threshold and the output of the female-related re-scaler is made for determining whether a female subject has nephrolithiasis. Then, the prediction model may output a result indicating whether the subject has nephrolithiasis or not based on the comparison between the common threshold and the output of the male-related or female-related re-scaler. Specifically, when the output of the male- or female-related re-scaler is greater than the common threshold, the prediction model may output a result indicating that the subject has nephrolithiasis. In some embodiments, the prediction model directly outputs the output of the male-related or female-related re-scaler (i.e., a numerical value ranging from zero to one) as a reference for risk assessment.

It is worth to note that in some embodiments, step S04 for re-grouping the training data sets into the male-related group and the female-related group, step S05 for determining the male-related re-scaler, step S06 for determining the female-related re-scaler, and the step of concatenating the average model, the male-related re-scaler and the female-related re-scaler in step S07 are omitted. That is to say, the average model obtained in step S03 is directly used as the prediction model for determining whether the subject has nephrolithiasis.

After the prediction model is obtained, the prediction model may be put to use in determining whether a subject has nephrolithiasis, where an input variable set that is related to the subject is fed into the prediction model so as to obtain an output indicating whether the subject has nephrolithiasis. Similar to each of the training data sets, the input variable set includes eight parameters, i.e., a gender indicator that indicates gender of the subject (e.g., one for male and zero for female), age of the subject, a BMI that is related to the subject, an eGFR that is related to the subject, a value of urine pH that is related to the subject, a gout indicator that indicates whether the subject was ever diagnosed with gout (e.g., one for having been diagnosed with gout and zero for never having been diagnosed with gout), a DM indicator that indicates whether the subject was ever diagnosed with DM (e.g., one for having been diagnosed with DM and zero for never having been diagnosed with DM), and a bacteriuria indicator that indicates whether the subject was ever diagnosed with bacteriuria (e.g., one for having been diagnosed with bacteriuria and zero for never having been diagnosed with bacteriuria). The eGFR is calculated by using the isotope dilution mass spectrometry traceable Modification of Diet in Renal Disease formula that is expressed as:

$$\text{eGFR } [mL/min/1.73m^2] = 175{\times}(Scr)^{-1.154}{\times}(Age)^{-0.203}{\times}(0.742)^G,$$

where *Scr* represents a blood creatinine concentration that is related to the subject, *Age* represents the age of the subject, and *G* represents the gender of the subject, has a value of one when the gender indicator indicates that the subject is female, and has a value of zero when the gender indicator indicates that the subject is male.

In one embodiment, the input variable set includes ten parameters, i.e., a number of red blood cells in a urine sample of the subject, a blood creatinine concentration that is related to the subject, the gender indicator that indicates gender of the subject, the age of the subject, the BMI that is related to the subject, the eGFR that is related to the subject, the value of urine pH that is related to the subject, the gout indicator that indicates whether the subject was ever diagnosed with gout, the DM indicator that indicates whether the subject was ever diagnosed with DM, and the bacteriuria indicator that indicates whether the subject was ever diagnosed with bacteriuria.

In one embodiment, the input variable set includes only three parameters, i.e., the gender indicator that indicates gender of the subject, the age of the subject and the eGFR that is related to the subject.

In one embodiment, the input variable set includes only three parameters, i.e., the gender indicator that indicates gender of the subject, the age of the subject and the blood creatinine concentration that is related to the subject.

In one embodiment, the input variable set includes only three parameters, i.e., the gender indicator that indicates gender of the subject, the age of the subject and the number of red blood cells in a urine sample of the subject.

It is worth to note that for each of the input variable set and the training data sets, the eGFR can be computed by manual calculation based on the blood creatinine concentration or can be determined by the prediction model when said each of the input variable set and the training data sets is fed into the prediction model.

In order to validate the prediction model obtained using the method according to the disclosure, data collected from Kaohsiung Medical University Hospital (KMUH), Kaohsiung Municipal Ta-Tung hospital (KMTTH) and Kaohsiung Municipal Siaogang Hospital (KMSH) was used. The data is related to 10813 patients, 2307 (accounting for 21.34% of the total) of whom have been diagnosed with nephrolithiasis and 8506 (accounting for 78.66% of the total) of whom were determined as not having nephrolithiasis after diagnosis. The data was grouped into training data sets, validation data sets and testing data sets. The training data sets are related to 5284 patients, 1114 (accounting for 21.08% of the total) of whom have been diagnosed with nephrolithiasis and 4170 (accounting for 78.92% of the total) of whom were determined as not having nephrolithiasis after diagnosis. The validation data sets are related to 1763 patients, 372 (accounting for 21.10% of the total) of whom have been diagnosed with nephrolithiasis and 1391 (accounting for 78.90% of the total) of whom were determined as not having nephrolithiasis after diagnosis. The testing data sets are related to 3766 patients, 821 (accounting for 21.80% of the total) of whom have been diagnosed with nephrolithiasis and 2945 (accounting for 78.20% of the total) of whom were determined as not having nephrolithiasis after diagnosis. Statistical details of clinical information related to the patients in the training data sets, the validation data sets and the testing data sets are summarized in Tables 2a, 2b, 2c, 3a, 3b and 3c below.

Table 2a

| | With Nephrolithiasis 1114 (21.08%) | Without Nephrolithiasis 4170 (78.92%) | Total 5284 (100%) | p-Value |
|---|---|---|---|---|
| Training Data Sets (48.87% of all Patients) | | | | |
| **Gender** | | | | <0.001 |
| Male | 756 (67.86%) | 3567 (85.54%) | 4323 (81.81%) | |
| Female | 358 (32.14%) | 603 (14.46%) | 961 (18.19%) | |
| **Age** | | | | <0.001 |

(continued)

| Training Data Sets (48.87% of all Patients) | | | |
|---|---|---|---|
| | With Nephrolithiasis | Without Nephrolithiasis | Total | p-Value |
| | 1114 (21.08%) | 4170 (78.92%) | 5284 (100%) | |
| ≤45 | 256 (23.79%) | 929 (22.28%) | 1194 (22.60%) | |
| 45-65 | 619 (55.57%) | 3229 (77.43%) | 3848 (72.82%) | |
| >65 | 230 (20.64%) | 12 (0.29%) | 242 (4.58%) | |
| **DM** | | | | <0.001 |
| With | 184 (16.52%) | 316 (7.58%) | 500 (9.46%) | |
| Without | 930 (83.48%) | 3854 (92.42%) | 4784 (90.54%) | |
| **Gout** | | | | <0.001 |
| With | 42 (3.77%) | 43 (1.03%) | 85 (1.61%) | |
| Without | 1072 (96.23%) | 4127 (98.97%) | 5199 (98.39%) | |
| **Bacteriuria** | | | | <0.001 |
| With | 160 (14.36%) | 191 (4.58%) | 351 (6.64%) | |
| Without | 954 (85.64%) | 3979 (95.42%) | 4933 (93.36%) | |

Table 2b

| Validation Data Sets (16.3% of all Patients) | | | |
|---|---|---|---|
| | With Nephrolithiasis | Without Nephrolithiasis | Total | p-Value |
| | 372 (21.10%) | 1391 (78.90%) | 1763 (100%) | |
| **Gender** | | | | <0.001 |
| Male | 234 (62.90%) | 1185 (85.19%) | 1419 (80.49%) | |
| Female | 138 (37.10%) | 206 (14.81%) | 344 (19.51%) | |
| **Age** | | | | 0.21 |
| ≤45 | 98 (26.34%) | 305 (21.92%) | 403 (22.86%) | |
| 45-65 | 197 (52.96%) | 1082 (77.79%) | 1279 (72.55%) | |
| >65 | 77 (20.70%) | 4 (0.29%) | 81 (4.59%) | |
| **DM** | | | | <0.001 |
| With | 61 (16.40%) | 108 (7.76%) | 169 (9.59%) | |
| Without | 311 (83.60%) | 1283 (92.24%) | 1594 (90.41%) | |
| Gout | | | | <0.001 |
| With | 16 (4.30%) | 13 (0.93%) | 29 (1.64%) | |
| Without | 356 (95.70%) | 1378 (99.07%) | 1734 (98.36%) | |
| **Bacteriuria** | | | | <0.001 |
| With | 55 (14.78%) | 65 (4.67%) | 120 (6.81%) | |
| Without | 317 (85.22%) | 1326 (95.33%) | 1643 (93.19%) | |

Table 2c

| Testing Data Sets (34.83% of all Patients) | | | |
|---|---|---|---|
| | With Nephrolithiasis | Without Nephrolithiasis | Total | p-Value |
| | 821 (21.8%) | 2945 (78.2%) | 3766 (100%) | |
| **Gender** | | | | 0.053 |
| Male | 513 (62.48%) | 1947 (66.11%) | 2460 (65.32%) | |
| Female | 308 (37.52%) | 998 (33.89%) | 1306 (34.68%) | |

(continued)

| Testing Data Sets (34.83% of all Patients) | | | |
|---|---|---|---|
| With Nephrolithiasis | Without Nephrolithiasis | Total | p-Value |
| 821 (21.8%) | 2945 (78.2%) | 3766 (100%) | |
| **Age** | | | <0.001 |
| ≤45 | 137 (16.68%) | 1340 (45.50%) | 1477 (39.22%) |
| 45-65 | 381 (46.41%) | 1543 (52.39%) | 1924 (51.09%) |
| >65 | 303 (36.91%) | 12 (2.11 %) | 242 (9.69%) |
| **DM** | | | <0.001 |
| With | 207 (25.21%) | 128 (4.35%) | 335 (8.90%) |
| Without | 614 (74.79%) | 2817 (95.65%) | 3431 (91.10%) |
| **Gout** | | | <0.001 |
| With | 39 (4.75%) | 24 (0.81 %) | 63 (1.67%) |
| Without | 782 (95.25%) | 2921 (99.19%) | 3703 (98.33%) |
| **Bacteriuria** | | | <0.001 |
| With | 95 (11.57%) | 224 (7.61%) | 319 (8.47%) |
| Without | 726 (88.43%) | 2721 (92.39%) | 3447 (91.53%) |

Table 3a

| Training Data Sets | | |
|---|---|---|
| With Nephrolithiasis | Without Nephrolithiasis | p-Value |
| **Age** | | <0.001 |
| Mean Standard | 55.16 | 53.54 |
| Deviation (SD) | 13.15 | 9.34 |
| 95 % Confidence | | |
| Interval (CI) | (54.39-55.93) | (53.26-53.83) |
| **BMI** | | <0.001 |
| Mean Standard | 25.64 | 25.24 |
| Deviation (SD) | 3.74 | 3.39 |
| 95 % Confidence | | |
| Interval (CI) | (25.42-25.86) | (25.14-25.34) |
| **Urine PH** | | 0.0046 |
| Mean Standard | 6.05 | 6.12 |
| Deviation (SD) | 0.79 | 0.7 |
| 95 % Confidence | | |
| Interval (CI) | (6.01-6.10) | (6.10-6.14) |
| **eGFR** | | <0.001 |
| Mean Standard | 75.67 | 81.68 |
| Deviation (SD) | 29.73 | 15.50 |
| 95 % Confidence | | |
| Interval (CI) | (73.93-77.42) | (81.21-82.15) |

Table 3b

| Validation Data Sets | | |
|---|---|---|
| With Nephrolithiasis | Without Nephrolithiasis | p-Value |
| **Age** | | 0.2087 |
| Mean | 54.43 | 53.69 |
| Standard | | |

(continued)

| Validation Data Sets | | |
|---|---|---|
| | With Nephrolithiasis | Without Nephrolithiasis | p-Value |
| Deviation (SD) | 12.99 | 9.26 | |
| 95 % Confidence Interval (CI) | (53.11-55.75) | (53.20-54.17) | |
| **BMI** | | | 0.0055 |
| Mean | 25.81 | 25.24 | |
| Standard Deviation (SD) | 3.81 | 3.44 | |
| 95 % Confidence Interval (CI) | (25.42-26.20) | (25.06-25.42) | |
| **Urine PH** | | | 0.0105 |
| Mean | 6.03 | 6.13 | |
| Standard Deviation (SD) | 0.78 | 0.69 | |
| 95 % Confidence Interval (CI) | (5.95-6.10) | (6.10-6.17) | |
| **eGFR** | | | <0.001 |
| Mean | 75.14 | 82.29 | |
| Standard Deviation (SD) | 30.36 | 15.84 | |
| 95 % Confidence Interval (CI) | (72.05-78.22) | (81.45-83.12) | |

Table 3c

| Testing Data Sets | | |
|---|---|---|
| | With Nephrolithiasis | Without Nephrolithiasis | p-Value |
| **Age** | | | <0.001 |
| Mean | 59.54 | 47.45 | |
| Standard Deviation (SD) | 13.70 | 10.96 | |
| 95 % Confidence Interval (CI) | (58.60-60.48) | (47.05-47.84) | |
| **BMI** | | | <0.001 |
| Mean | 26.36 | 24.51 | |
| Standard Deviation (SD) | 5.0 | 3.76 | |
| 95 % Confidence Interval (CI) | (26.01-26.70) | (24.37-24.65) | |
| **Urine PH** | | | 0.0299 |
| Mean | 6.13 | 6.07 | |
| Standard Deviation (SD) | 0.81 | 0.73 | |
| 95 % Confidence Interval (CI) | (6.08-6.19) | (6.04-6.09) | |
| **eGFR** | | | <0.001 |
| Mean | 68.15 | 88.07 | |
| Standard Deviation (SD) | 26.70 | 17.61 | |

(continued)

| | Testing Data Sets | | |
| --- | --- | --- | --- |
| | With Nephrolithiasis | Without Nephrolithiasis | p-Value |
| 95 % Confidence Interval (CI) | (66.32-69.98) | (87.43-88.70) | |

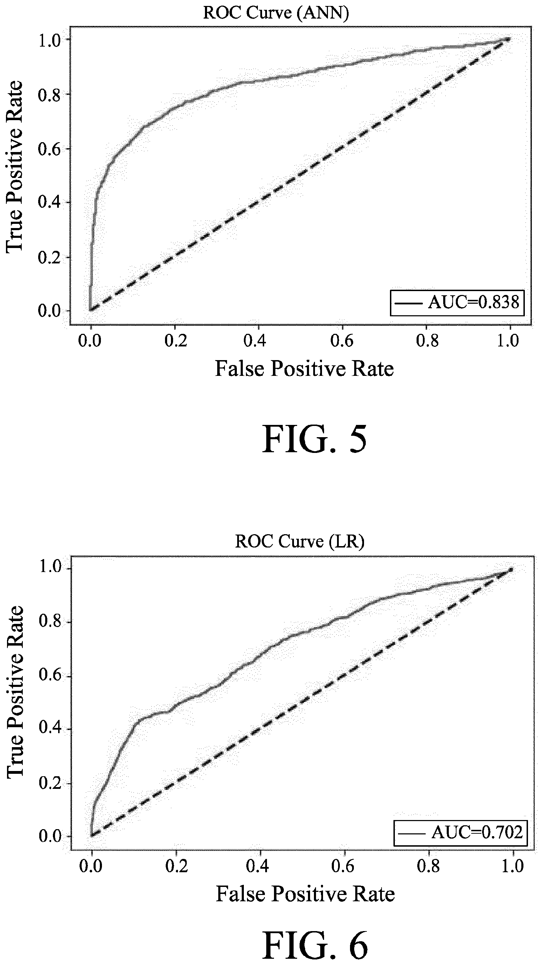

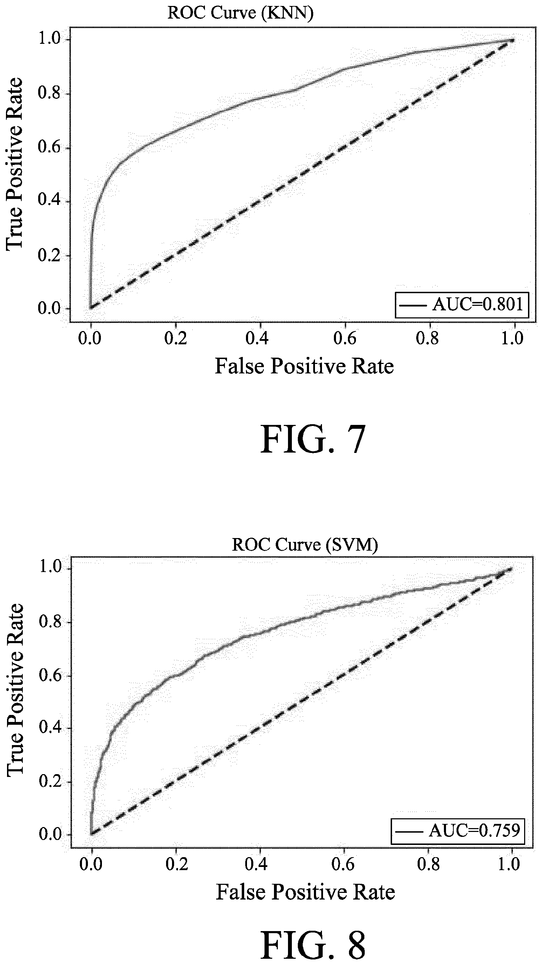

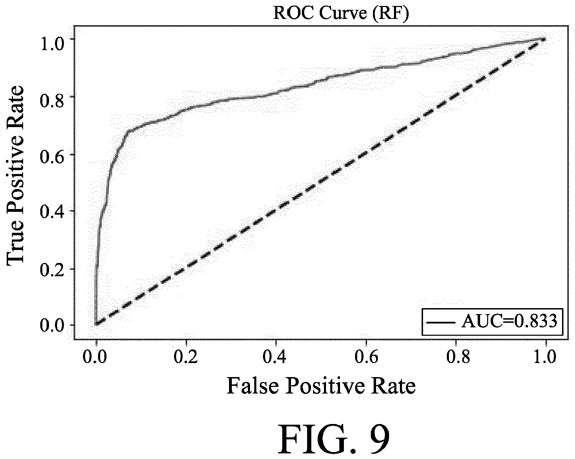

Moreover, referring to Tables 4a, 4b and 4c below, performance of the prediction model established by using the method according to the disclosure, which is denoted by "ANN" in Tables 4a, 4b and 4c, is compared to performance of other conventional models established by using algorithms of logistic regression (LR), k-nearest neighbor (KNN), support vector machine (SVM) and random forest (RF), which are denoted respectively by "LR", "KNN", "SVM" and "RF" in Tables 4a, 4b and 4c. It is worth to note that for each of the aforementioned conventional models, an optimal cut-off threshold was determined by applying the conventional model to the training data sets, and then the optimal cut-off threshold thus determined was used for the validation data sets and the testing data sets. For the prediction model established by using the method according to the disclosure, accuracies of 80% and 75.7% were achieved respectively for the validation data sets and the testing data sets. The prediction model (i.e., an ANN model) achieved relatively higher accuracies than the conventional models established by using algorithms of LR, KNN, SVM and RF (hereinafter also referred to as LR, KNN, SVM and RF models, respectively). Furthermore, ROC curves for the testing data sets are plotted in Figures 5 to 9 respectively for the prediction model and the LR, KNN, SVM and RF models. An area under the curve (AUC) achieved by using the prediction model is greater than those achieved by using the LR, KNN, SVM and RF models.

Table 4a

| Model | Data Set | AUC (95% CI) | Optimal Cut-off Threshold | Accuracy (95% CI) |
| --- | --- | --- | --- | --- |
| ANN | Training | 0.903 (0.8907-0.9153) | 0.5 | 0.828 (0.8174-0.8378) |
| | Validation | 0.869 (0.8448-0.8932) | *** | 0.800 (0.7811-0.8185) |
| | Testing | 0.838 (0.8203-0.8557) | *** | 0.757 (0.7433-0.7707) |
| LR | Training | 0.660 (0.6410-0.6790) | 0.49 | 0.710 (0.6980-0.7225) |
| | Validation | 0.691 (0.6587-0.7233) | *** | 0.710 (0.6890-0.7313) |
| | Testing | 0.702 (0.6804-0.7236) | *** | 0.618 (0.6029-0.6339) |
| KNN | Training | 0.904 (0.8918-0.9162) | 0.14 | 0.779 (0.7680-0.7903) |
| | Validation | 0.862 (0.8373-0.8867) | *** | 0.766 (0.7460-0.7855) |
| | Testing | 0.801 (0.7819-0.8201) | *** | 0.581 (0.5647-0.5962) |
| SVM | Training | 0.645 (0.6259-0.6641) | 0.24 | 0.775 (0.7635-0.7861) |
| | Validation | 0.687 (0.6547-0.7193) | *** | 0.785 (0.7653-0.8037) |
| | Testing | 0.759 (0.7386-0.7794) | *** | 0.734 (0.7196-0.7478) |
| RF | Training | 0.871 (0.8571-0.8849) | 0.21 | 0.827 (0.8172-0.8376) |
| | Validation | 0.865 (0.8405-0.8895) | *** | 0.819 (0.8005-0.8365) |
| | Testing | 0.833 (0.8151-0.8509) | *** | 0.736 (0.7220-0.7501) |

Table 4b

| Model | Data Set | Sensitivity (95% CI) | Specificity (95% CI) |
| --- | --- | --- | --- |
| ANN | Training | 0.803 (0.7791-0.8259) | 0.834 (0.8230-0.8456) |
| | Validation | 0.742 (0.6975-0.7864) | 0.815 (0.7948-0.8356) |
| | Testing | 0.780 (0.7512-0.8079) | 0.751 (0.7351-0.7664) |

(continued)

| Model | Data Set | Sensitivity (95% CI) | Specificity (95% CI) |
|---|---|---|---|
| LR | Training | 0.555 (0.5256-0.5839) | 0.752 (0.7387-0.7649) |
| | Validation | 0.575 (0.5250-0.6255) | 0.746 (0.7234-0.7691) |
| | Testing | 0.669 (0.6365-0.7009) | 0.604 (0.5868-0.6221) |
| KNN | Training | 0.883 (0.8644-0.9022) | 0.751 (0.7382-0.7644) |
| | Validation | 0.825 (0.7867-0.8639) | 0.750 (0.7271-0.7726) |
| | Testing | 0.812 (0.7857-0.8391) | 0.516 (0.4977-0.5338) |
| SVM | Training | 0.512 (0.4823-0.5410) | 0.845 (0.8341-0.8561) |
| | Validation | 0.551 (0.5005-0.6016) | 0.847 (0.8279-0.8658) |
| | Testing | 0.625 (0.5917-0.6580) | 0.764 (0.7487-0.7793) |
| RF | Training | 0.709 (0.6825-0.7358) | 0.859 (0.8484-0.8696) |
| | Validation | 0.718 (0.6720-0.7635) | 0.845 (0.8264-0.8644) |
| | Testing | 0.783 (0.7550-0.8114) | 0.723 (0.7068-0.7391) |

Table 4c

| Model | Data Set | Positive Predictive Value (PPV) (95% CI) | Negative Predictive Value (NPV) (95% CI) |
|---|---|---|---|
| ANN | Training | 0.564 (0.5396-0.5885) | 0.941 (0.9329-0.9481) |
| | Validation | 0.518 (0.4754-0.5602) | 0.922 (0.9070-0.9369) |
| | Testing | 0.466 (0.4394-0.4922) | 0.924 (0.9137-0.9349) |
| LR | Training | 0.374 (0.3505-0.3972) | 0.863 (0.8522-0.8746) |
| | Validation | 0.377 (0.3375-0.4173) | 0.868 (0.8487-0.8871) |
| | Testing | 0.320 (0.2982-0.3424) | 0.867 (0.8528-0.8821) |
| KNN | Training | 0.487 (0.4651-0.5087) | 0.960 (0.9534-0.9669) |
| | Validation | 0.469 (0.4305-0.5069) | 0.941 (0.9275-0.9552) |
| | Testing | 0.319 (0.2987-0.3386) | 0.908 (0.8941-0.9218) |
| SVM | Training | 0.469 (0.4407-0.4968) | 0.866 (0.8558-0.8767) |
| | Validation | 0.490 (0.4425-0.5384) | 0.876 (0.8582-0.8935) |
| | Testing | 0.425 (0.3968-0.4525) | 0.880 (0.8670-0.8922) |
| RF | Training | 0.573 (0.5472-0.5994) | 0.917 (0.9084-0.9257) |
| | Validation | 0.554 (0.5096-0.5983) | 0.918 (0.9030-0.9331) |
| | Testing | 0.441 (0.4152-0.4662) | 0.923 (0.9120-0.9337) |

Tables 5a, 5b and 5c below show performance of the prediction model in a scenario where only three parameters are contained in each of the training data sets, the validation data sets and the testing data sets. For Table 5a, the three parameters contained in each of the training data sets, the validation data sets and the testing data sets are the gender indicator, the age and the eGFR. For Table 5b, the three parameters contained in each of the training data sets, the validation data sets and the testing data sets are the gender indicator, the age and the blood creatinine concentration. For Table 5c, the three parameters contained in each of the training data sets, the validation data sets and the testing data sets are the gender indicator, the age and the number of red blood cells in a urine sample.

Table 5a

| Data Set | AUC | Accuracy | Sensitivity | Specificity |
|---|---|---|---|---|
| Training | 0.832 | 0.7829 | 0.6943 | 0.8268 |
| Validation | 0.809 | 0.7562 | 0.6618 | 0.8029 |
| Testing | 0.81 | 0.7466 | 0.7225 | 0.7551 |

Table 5b

| Data Set | AUC | Accuracy | Sensitivity | Specificity |
|---|---|---|---|---|
| Training | 0.818 | 0.7930 | 0.6203 | 0.8785 |
| Validation | 0.786 | 0.7602 | 0.5845 | 0.8471 |
| Testing | 0.819 | 0.8063 | 0.6450 | 0.8638 |

Table 5c

| Data Set | AUC | Accuracy | Sensitivity | Specificity |
|---|---|---|---|---|
| Training | 0.936 | 0.9105 | 0.8479 | 0.9415 |
| Validation | 0.931 | 0.8881 | 0.8285 | 0.9176 |
| Testing | 0.923 | 0.8871 | 0.8225 | 0.9101 |

To sum up, in the method for establishing a model to determine whether a subject has nephrolithiasis according to the disclosure, the prediction model is built by concatenating the average of the preliminary models that are trained by using the N-fold cross-validation protocol, and the male-related re-scaler and the female-related re-scaler that are determined respectively for male and female subjects. Since male and female subjects are diagnosed respectively according to the male-related cut-off threshold and the female-related cut-off threshold, accuracy of diagnosis made by using the prediction model may be improved. Moreover, convenience of using the prediction model is ensured because minimal clinical information is needed to serve as the input of the prediction model, including gender, age, BMI, eGFR, urine pH level, and medical history about gout, DM and bacteriuria of a subject, which is easily obtainable.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

Claims

1. A method for establishing a model to determine whether a subject has nephrolithiasis, **characterized by**:

   grouping a plurality of training data sets that are respectively related to a plurality of patients into a number N of preliminary groups, where N is a positive integer;
   obtaining a number N of preliminary models based on the preliminary groups;

averaging the preliminary models to obtain an average model; and
obtaining, based on the average model, a prediction model for determining whether the subject has nephrolithiasis.

2. The method as claimed in claim 1, wherein:

each of the preliminary models is expressed as $M_i(x) = \dfrac{1}{1+e^{f_i(x)}}$ , $i$ = 1,2, ... , N, where $M_i$ represents the preliminary model, $x$ represents an input of the preliminary model, and $f_i(x)$ is a nonlinear function that is obtained by using a multi-layer fully connected neural network; and
the multi-layer fully connected neural network has a plurality of non-output layers, and each of the non-output layers consists of three concatenated components respectively for batch normalization, affine transformation and activation.

3. The method as claimed in one of claims 1 and 2, further comprising:

re-grouping the training data sets into a male-related group and a female-related group, the male-related group including those of the training data sets that are related to those of the patients who are male, the female-related group including those of the training data sets that are related to those of the patients who are female;
applying the average model to the training data sets included in the male-related group to obtain a plurality of male-related outputs, and then obtaining a male-related receiver operating characteristic, ROC, curve based on the male-related outputs;
determining a male-related cut-off threshold based on the male-related ROC curve;
determining a male-related re-scaler based on the male-related cut-off threshold;
applying the average model to the training data sets included in the female-related group to obtain a plurality of female-related outputs, and then obtaining a female-related ROC curve based on the female-related outputs;
determining a female-related cut-off threshold based on the female-related ROC curve;
determining a female-related re-scaler based on the female-related cut-off threshold; and
concatenating the average model, the male-related re-scaler and the female-related re-scaler to obtain the prediction model for determining whether the subject has nephrolithiasis.

4. The method as claimed in any one of claims 1 to 3, wherein each of the training data sets includes a gender indicator that indicates gender of the respective one of the patients, age of the respective one of the patients, and an estimated glomerular filtration rate, eGFR, that is related to the respective one of the patients.

5. The method as claimed in claim 4, wherein each of the training data sets further includes one of a number of red blood cells in a urine sample of the respective one of the patients, a blood creatinine concentration that is related to the respective one of the patients, a body mass index, BMI, that is related to the respective one of the patients, a value of urine pH that is related to the respective one of the patients, a gout indicator that indicates whether the respective one of the patients was ever diagnosed with gout, a diabetes mellitus, DM, indicator that indicates whether the respective one of the patients was ever diagnosed with DM, a bacteriuria indicator that indicates whether the respective one of the patients was ever diagnosed with bacteriuria, and any combination thereof.

6. The method as claimed in any one of claims 1 to 3, wherein each of the training data sets includes a gender indicator that indicates gender of the respective one of the patients, age of the respective one of the patients, and a blood creatinine concentration that is related to the respective one of the patients.

7. The method as claimed in claim 6, wherein each of the training data sets further includes one of a number of red blood cells in a urine sample of the respective one of the patients, an estimated glomerular filtration rate, eGFR, that is related to the respective one of the patients, a body mass index, BMI, that is related to the respective one of the patients, a value of urine pH that is related to the respective one of the patients, a gout indicator that indicates whether the respective one of the patients was ever diagnosed with gout, a diabetes mellitus, DM, indicator that indicates whether the respective one of the patients was ever diagnosed with DM, a bacteriuria indicator that indicates whether the respective one of the patients was ever diagnosed with bacteriuria, and any combination thereof.

8. The method as claimed in any one of claims 1 to 3, wherein each of the training data sets includes a gender indicator that indicates gender of the respective one of the patients, age of the respective one of the patients, and a number of red blood cells in a urine sample of the respective one of the patients.

9. The method as claimed in claim 8, wherein each of the training data sets further includes one of an estimated glomerular filtration rate, eGFR, that is related to the respective one of the patients, a blood creatinine concentration that is related to the respective one of the patients, a body mass index, BMI, that is related to the respective one of the patients, a value of urine pH that is related to the respective one of the patients, a gout indicator that indicates whether the respective one of the patients was ever diagnosed with gout, a diabetes mellitus, DM, indicator that indicates whether the respective one of the patients was ever diagnosed with DM, a bacteriuria indicator that indicates whether the respective one of the patients was ever diagnosed with bacteriuria, and any combination thereof.

10. The method as claimed in any one of claims 1 to 9, further comprising feeding an input variable set into the prediction model so as to obtain an output indicating whether the subject has nephrolithiasis, the input variable set being related to the subject.

11. The method as claimed in claim 10, wherein the input variable set includes a gender indicator that indicates gender of the subject, age of the subject, and an estimated glomerular filtration rate, eGFR, that is related to the subject.

12. The method as claimed in claim 11, wherein the input variable set further includes one of a number of red blood cells in a urine sample of the subject, a blood creatinine concentration that is related to the subject, a body mass index, BMI, that is related to the subject, a value of urine pH that is related to the subject, a gout indicator that indicates whether the subject was ever diagnosed with gout, a diabetes mellitus, DM, indicator that indicates whether the subject was ever diagnosed with DM, a bacteriuria indicator that indicates whether the subject was ever diagnosed with bacteriuria, and any combination thereof.

13. The method as claimed in claim 10, wherein the input variable set includes a gender indicator that indicates gender of the subject, age of the subject, and a blood creatinine concentration that is related to the subject.

14. The method as claimed in claim 13, wherein the input variable set further includes one of a number of red blood cells in a urine sample of the subject, an estimated glomerular filtration rate, eGFR, that is related to the subject, a body mass index, BMI, that is related to the subject, a value of urine pH that is related to the subject, a gout indicator that indicates whether the subject was ever diagnosed with gout, a diabetes mellitus, DM, indicator that indicates whether the subject was ever diagnosed with DM, a bacteriuria indicator that indicates whether the subject was ever diagnosed with bacteriuria, and any combination thereof.

15. The method as claimed in claim 10, wherein the input variable set includes a gender indicator that indicates gender of the subject, age of the subject, and a number of red blood cells in a urine sample of the subject.

16. The method as claimed in claim 15, wherein the input variable set further includes one of a blood creatinine concentration that is related to the subject, an estimated glomerular filtration rate, eGFR, that is related to the subject, a body mass index, BMI, that is related to the subject, a value of urine pH that is related to the subject, a gout indicator that indicates whether the subject was ever diagnosed with gout, a diabetes mellitus, DM, indicator that indicates whether the subject was ever diagnosed with DM, a bacteriuria indicator that indicates whether the subject was ever diagnosed with bacteriuria, and any combination thereof.

Grouping training data sets into preliminary groups — S01

Obtaining preliminary models by using
N-fold cross-validation protocol based
on preliminary groups — S02

Averaging preliminary models to obtain average model — S03

Re-grouping training data sets into male-related
group and female-related group — S04

S05

Obtaining male-related ROC curve — S51

Obtaining male-related cut-off threshold — S52

Determining male-related re-scaler — S53

S06

Obtaining female-related ROC curve — S61

Obtaining female-related cut-off threshold — S62

Determining female-related re-scaler — S63

Concatenating average model, male-related
re-scaler and female-related re-scaler to
obtain prediction model — S07

# FIG. 1

Input → Batch normalization → Affine transformation → Activation → Output

# FIG. 2

Input → Batch normalization → Affine transformation → Output

# FIG. 3

FIG. 4

ROC Curve (ANN)

FIG. 5

ROC Curve (LR)

FIG. 6

FIG. 7

FIG. 8

FIG. 9

Europäisches
Patentamt

European
Patent Office

Office européen
des brevets

# EUROPEAN SEARCH REPORT

Application Number

EP 23 21 9076

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAO-WEI CHEN: "Prediction of the Uric Acid Component in Nephrolithiasis Using Simple Clinical Information about Metabolic Disorder and Obesity: A Machine Learning-Based Model", NUTRIENTS, vol. 14, no. 9, 27 April 2022 (2022-04-27), page 1829, XP093160636, CH ISSN: 2072-6643, DOI: 10.3390/nu14091829 * abstract; figure 1; tables 1-3 * * page 2, paragraph 2 – page 7, paragraph 4 * | 1-16 | INV. G16H50/20 |
| X | US 2020/005900 A1 (CHA THEODORE [US] ET AL) 2 January 2020 (2020-01-02) * abstract * * paragraph [0024] – paragraph [0034] * * paragraph [0075] – paragraph [0104] * | 1-16 | |
| X | EP 2 743 852 A1 (UNI AUT NOMA DE BARCELONA [ES]; MASARYK UNIVERSITY [CZ]) 18 June 2014 (2014-06-18) * abstract * * paragraph [0001] * * paragraph [0013] – paragraph [0027] * | 1-16 | TECHNICAL FIELDS SEARCHED (IPC) G16H |
| A | Anonymous: "Ensemble averaging (machine learning) – Wikipedia", , 20 February 2020 (2020-02-20), pages 1-3, XP055930108, Retrieved from the Internet: URL:https://web.archive.org/web/20200220180211/https://en.wikipedia.org/wiki/Ensemble_averaging_(machine_learning) [retrieved on 2022-06-10] * the whole document * | 1-16 | |

The present search report has been drawn up for all claims

| Place of search | Date of completion of the search | Examiner |
|---|---|---|
| Munich | 10 May 2024 | Menschner, Philipp |

## ANNEX TO THE EUROPEAN SEARCH REPORT
## ON EUROPEAN PATENT APPLICATION NO.

EP 23 21 9076

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2024

| Patent document cited in search report | Publication date | Patent family member(s) | Publication date |
|---|---|---|---|
| US 2020005900 A1 | 02-01-2020 | US 2020005900 A1<br>US 2021183471 A1<br>WO 2020006571 A1 | 02-01-2020<br>17-06-2021<br>02-01-2020 |
| EP 2743852 A1 | 18-06-2014 | EP 2743852 A1<br>WO 2013017725 A1 | 18-06-2014<br>07-02-2013 |

For more details about this annex : see Official Journal of the European Patent Office, No. 12/82